# EUROPEAN PATENT SPECIFICATION

(11) **EP 4 389 025 B1**
(45) Date of publication and mention of the grant of the patent: **10.12.2025**
(21) Application number: 23198867.6
(22) Date of filing: 21.09.2023
(51) Int. Cl.: A61B 17/32, A61B 18/14, A61F 2/24, A61B 17/22, A61B 17/295

(54) **TRANSCATHETER TOOLS FOR BASILICA OR LAMPOON PROCEDURE**
TRANSKATHETERWERKZEUGE FÜR BASILICA- ODER LAMPOON-VERFAHREN
OUTILS TRANSCATHÉTER POUR PROCÉDURE BASILICE OU LABOON

(30) Priority: 28.11.2022 US 202263385061 P
(43) Date of publication of application: 26.06.2024
(73) Proprietor: St. Jude Medical, Cardiology Division, Inc., St. Paul MN 55117 (US)
(72) Inventor: STEENWYK, Nicholas, Minneapolis, MN (US)
(74) Representative: Gill Jennings & Every LLP

(56) References cited:
- WO-A1-2022/013857
- US-A- 5 626 578
- US-A1- 2020 146 691
- US-A1- 2021 137 579
- US-A1- 2021 330 355
- US-A1- 2021 346 045
- US-A1- 2022 233 210

## Description

### Cross-Reference to Related Applications

The present application claims priority to U.S. Provisional Serial No. 63/385,061, filed November 28, 2022.

### Background of the Disclosure

Valvular heart disease, and specifically aortic and mitral valve disease, is a significant health issue in the United States. Valve replacement is one option for treating heart valve diseases. Prosthetic heart valves, including surgical heart valves and collapsible/expandable heart valves intended for transcatheter aortic valve replacement ("TAVR") or transcatheter mitral valve replacement ("TMVR"), are well known in the patent literature. Surgical or mechanical heart valves may be sutured into a native annulus of a patient during an open-heart surgical procedure, for example. Collapsible/expandable heart valves may be delivered into a patient via a tube-like delivery apparatus such as a catheter, a trocar, a laparoscopic instrument, or the like to avoid a more invasive procedure such as full open-chest, open-heart surgery. As used herein, reference to a "collapsible/expandable" heart valve includes heart valves that are formed with a small cross-section that enables them to be delivered into a patient through a tube-like delivery apparatus in a minimally invasive procedure, and then expanded to an operable state once in place, as well as heart valves that, after construction, are first collapsed to a small cross-section for delivery into a patient and then expanded to an operable size once in place in the valve annulus.

Collapsible/expandable prosthetic heart valves typically take the form of a one-way valve structure (often referred to herein as a valve assembly) mounted to/within an expandable stent. In general, these collapsible/expandable heart valves include a self-expanding or balloon-expandable stent, often made of nitinol or another shape-memory metal or metal alloy (for self-expanding stents) or steel or cobalt chromium (for balloon-expandable stents). Existing collapsible/expandable TAVR devices have been known to use different configurations of stent layouts - including straight vertical struts connected by "V"s as illustrated in U.S. Pat. No. 8,454,685, or diamond-shaped cell layouts as illustrated in U.S. Pat. No. 9,326,856. The one-way valve assembly mounted to/within the stent includes one or more leaflets, and may also include a cuff or skirt. The cuff may be disposed on the stent's interior or luminal surface, its exterior or abluminal surface, and/or on both surfaces. A cuff helps to ensure that blood does not flow around the valve leaflets if the valve or valve assembly is not optimally seated in a valve annulus. A cuff, or a portion of a cuff, disposed on the exterior of the stent can help retard leakage around the outside of the valve (the latter known as paravalvular or "PV" leakage).

Balloon expandable valves are typically delivered to the native annulus while collapsed (or "crimped") onto a deflated balloon of a balloon catheter, with the collapsed valve being either covered or uncovered by an overlying sheath. Once the crimped prosthetic heart valve is positioned within the annulus of the native heart valve that is being replaced, the balloon is inflated to force the balloon expandable valve to transition from the collapsed or crimped condition into an expanded or deployed condition, with the prosthetic heart valve tending to remain in the shape into which it is expanded by the balloon. Typically, when the position of the collapsed prosthetic heart valve is determined to be in the desired position relative to the native annulus (*e.g.* via visualization under fluoroscopy), a fluid (typically a liquid although gas could be used as well) such as saline is pushed via a syringe (manually, automatically, or semi-automatically) through the balloon catheter to cause the balloon to begin to fill and expand, and thus cause the overlying prosthetic heart valve to expand into the native annulus.

When self-expandable prosthetic heart valves are delivered into a patient to replace a malfunctioning native heart valve, the self-expandable prosthetic heart valve is almost always maintained in the collapsed condition within a capsule of the delivery device. While the capsule may ensure that the prosthetic heart valve does not self-expand prematurely, the overlying capsule (with or without the help of additional internal retaining features) helps ensure that the prosthetic heart valve does not come into contact with any tissue prematurely, as well as helping to make sure that the prosthetic heart valve stays in the desired position and orientation relative to the delivery device during delivery. However, balloon expandable prosthetic heart valves are typically crimped onto the balloon of a delivery device without a separate capsule that overlies and/or protects the prosthetic heart valve. One reason for this is that space is always at a premium in transcatheter prosthetic heart valve delivery devices and systems, and adding a capsule in addition to the prosthetic valve and the underlying balloon may not be feasible given the size profile requirements of these procedures.

In some examples, such as when a prosthetic heart valve has failed, or has neared the end of its life cycle, a second prosthetic valve may be placed within the first prosthetic valve. Obstruction of one or more of the coronary arteries occurs in around 3% of such valve-in-valve cases and is associated with 50% in-hospital mortality. As younger patients are offered less invasive transcatheter valve replacements, rates of valve-in-valve procedures are likely to increase. Relatedly, physicians are increasingly focusing on lifetime management and preserving coronary access. However, coronary obstruction may occur in some valve-in-valve cases because the new valve frame pushes open leaflets of the previously implanted prosthetic valve forcing them in a permanently open position. In some anatomies, this causes the leaflets to obstruct or restrict flow into the coronary sinuses.

Some physicians have begun taking a new approach to valve-in-valve procedures to reduce the risk of coronary obstruction with a procedure now known as the "BASILICA" procedure, an acronym for Bioprosthetic or native Aortic Scallop Intentional Laceration to prevent Iatrogenic Coronary Artery obstruction. In the BASILICA procedure, the physician uses a series of conventional guide catheters, snares, and wires designed for general interventional procedures to lacerate the leaflet, creating a V-shaped opening when the new valve is deployed into the old valve. The BASILICA procedure is typically immediately followed by a second prosthetic valve implantation as it causes significant hemodynamic instability. Currently, there are no specialized tools or instruments to perform a simple and safe BASILICA procedure.

Among other advantages, it would be beneficial to provide new tools to achieve a consistent and simpler BASILICA procedures. WO 2022/013857 A1 relates to a valve-splitting device for splitting a native or prosthetic valve cusp before performing TAVI, in order to prevent coronary artery obstruction.

### Brief Summary of the Disclosure

According to the invention as defined by claim 1, a medical instrument includes a flexible sheath configured to pass through the femoral artery, an inner catheter at least partially disposed within the sheath and rotatable relative thereto, the inner catheter having a deflectable section, a pair of opposing jaws coupled to the inner catheter, the pair of opposing jaws having an open condition and a closed condition, and a cutting mechanism disposed adjacent to at least one of the pair of opposing jaws wherein the cutting mechanism comprises an electrosurgical wire passing through the inner catheter to a generator, and extending through a first of the pair of opposing jaws and toward a second of the pair of opposing jaws; and a conductive wire terminating in a conductive receptacle disposed in the second of the pair of opposing jaws, the conductive receptacle being coupleable with the electrosurgical wire to form an electrical circuit. Further embodiments of the invention are defined by the dependent claims.

In another example, a method of treatment not forming a part of the claimed invention includes providing a medical instrument including a flexible sheath configured to pass through the femoral artery, an inner catheter at least partially disposed within the sheath and rotatable relative thereto, the inner catheter having a deflectable section, a pair of opposing jaws coupled to the inner catheter, the pair of opposing jaws having an open condition and a closed condition, and a cutting mechanism disposed adjacent to at least one of the pair of opposing jaws, advancing the medical instrument to a prosthetic leaflet of a first prosthetic heart valve, grasping the prosthetic leaflet with the pair of opposing jaws, and cutting the prosthetic leaflet with the cutting mechanism.

### Brief Description of the Drawings

Fig. 1A is a perspective view of a stent of a prosthetic heart valve according to an embodiment of the disclosure.
Fig. 1B is a schematic front view of a section of the stent of Fig. 1A.
Fig. 1C is a schematic front view of a section of a stent according to an alternate embodiment of the prosthetic heart valve of Fig. 1A.
Figs. 1D-E are front views of the stent section of Fig. 1C in a collapsed and expanded state, respectively.
Figs. 1F-G are side views of a portion of the stent according to the embodiment of Fig. 1C in a collapsed and expanded state, respectively.
Fig. 1H is a flattened view of the stent according to the embodiment of Fig. 1C, as if cut and rolled flat.
Figs. 1I-J are front and side views, respectively, of a prosthetic heart valve including the stent of Fig. 1C.
Fig. 1K illustrates the view of Fig. 1H with an additional outer cuff provided on the stent.
Figs. 2A-B illustrates a prosthetic heart valve PHV, crimped over a balloon in the deflated and inflated conditions.
Figs. 3A-B are schematic front views showing valve-in-valve procedures.
Fig. 4 is a schematic logic flowchart to determine when a BASILICA procedure is appropriate.
Fig. 5 shows schematic illustrations of situations in which a BASILICA procedure may be appropriate.
Fig. 6 is a schematic illustration of a medical instrument for cutting a leaflet.
Figs. 7A-F are schematic side views of one example of a jaw assembly having a blade, and several variations of the cutting mechanism.
Fig. 8A is a schematic side view of an example of a jaw assembly have an electrosurgical wire, corresponding with the present claimed invention.
Fig. 8B is a schematic top view of lacerating a leaflet using the medical instrument of Fig. 8A.

### Detailed Description

As used herein, the term "inflow end" when used in connection with a prosthetic heart valve refers to the end of the prosthetic valve into which blood first enters when the prosthetic valve is implanted in an intended position and orientation, while the term "outflow end" refers to the end of the prosthetic valve where blood exits when the prosthetic valve is implanted in the intended position and orientation. Thus, for a prosthetic aortic valve, the inflow end is the end nearer the left ventricle while the outflow end is the end nearer the aorta. The intended position and orientation are used for the convenience of describing the valve disclosed herein, however, it should be noted that the use of the valve is not limited to the intended position and orientation, but may be deployed in any type of lumen or passageway. For example, although the prosthetic heart valve is described herein as a prosthetic aortic valve, the same or similar structures and features can be employed in other heart valves, such as the pulmonary valve, the mitral valve, or the tricuspid valve. Further, the term "proximal," when used in connection with a delivery device or system, refers to a direction relatively close to the user of that device or system when being used as intended, while the term "distal" refers to a direction relatively far from the user of the device. In other words, the leading end of a delivery device or system is positioned distal to a trailing end of the delivery device or system, when being used as intended. As used herein, the terms "substantially," "generally," "approximately," and "about" are intended to mean that slight deviations from absolute are included within the scope of the term so modified. As used herein, the stent may assume an "expanded state" and a "collapsed state," which refer to the relative radial size of the stent.

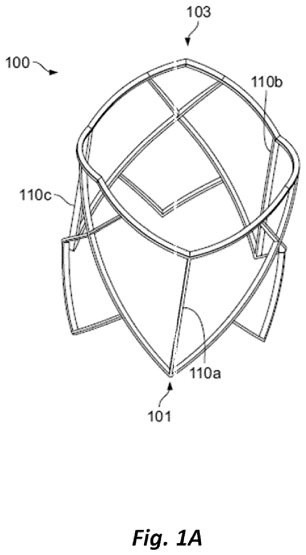

Fig. 1A illustrates a perspective view of a stent 100 of a prosthetic heart valve according to an embodiment of the disclosure. Stent 100 may include a frame extending in an axial direction between an inflow end 101 and an outflow end 103. Stent 100 includes three generally symmetric sections, wherein each section spans about 120 degrees around the circumference of stent 100. Stent 100 includes three vertical struts 110a, 110b, 110c, that extend in an axial direction substantially parallel to the direction of blood flow through the stent, which may also be referred to as a central longitudinal axis. Each vertical strut 110a, 110b, 110c may extend substantially the entire axial length between the inflow end 101 and the outflow end 103 of the stent 100, and may be disposed between and shared by two sections. In other words, each section is defined by the portion of stent 100 between two vertical struts. Thus, each vertical strut 110a, 110b, 110c is also separated by about 120 degrees around the circumference of stent 100. It should be understood that, if stent 100 is used in a prosthetic heart valve having three leaflets, the stent may include three sections as illustrated. However, in other embodiments, if the prosthetic heart valve has two leaflets, the stent may only include two of the sections.

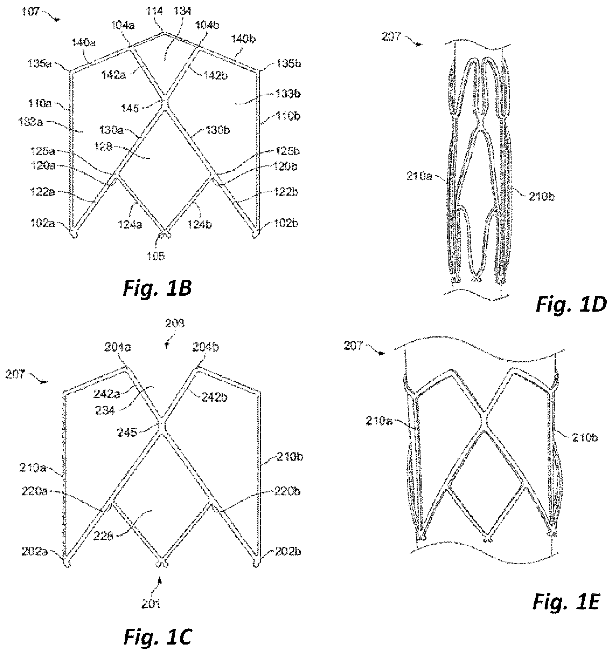

Fig. 1B illustrates a schematic view of a stent section 107 of stent 100, which will be described herein in greater detail and which is representative of all three sections. Stent section 107 depicted in Fig. 1B includes a first vertical strut 110a and a second vertical strut 110b. First vertical strut 110a extends axially between a first inflow node 102a and a first outer node 135a. Second vertical strut 110b extends axially between a second inflow node 102b and a second outer node 135b. As is illustrated, the vertical struts 110a, 110b may extend almost the entire axial length of stent 100. In some embodiments, stent 100 may be formed as an integral unit, for example by laser cutting the stent from a tube. The term "node" may refer to where two or more struts of the stent 100 meet one another. A pair of sequential inverted V's extends between inflow nodes 102a, 102b, which includes a first inflow inverted V 120a and a second inflow inverted V 120b coupled to each other at an inflow node 105. First inflow inverted V 120a comprises a first outer lower strut 122a extending between first inflow node 102a and a first central node 125a. First inflow inverted V 120a further comprises a first inner lower strut 124a extending between first central node 125a and inflow node 105. A second inflow inverted V 120b comprises a second inner lower strut 124b extending between inflow node 105 and a second central node 125b. Second inflow inverted V 120b further comprises a second outer lower strut 122b extending between second central node 125b and second inflow node 102b. Although described as inverted V's, these structures may also be described as half-cells, each half cell being a half-diamond cell with the open portion of the half-cell at the inflow end 101 of the stent 100.

Stent section 107 further includes a first central strut 130a extending between first central node 125a and an upper node 145. Stent section 107 also includes a second central strut 130b extending between second central node 125b and upper node 145. First central strut 130a, second central strut 130b, first inner lower strut 124a and second inner lower strut 124b form a diamond cell 128. Stent section 107 includes a first outer upper strut 140a extending between first outer node 135a and a first outflow node 104a. Stent section 107 further includes a second outer upper strut 140b extending between second outer node 135b and a second outflow node 104b. Stent section 107 includes a first inner upper strut 142a extending between first outflow node 104a and upper node 145. Stent section 107 further includes a second inner upper strut 142b extending between upper node 145 and second outflow node 104b. Stent section 107 includes an outflow inverted V 114 which extends between first and second outflow nodes 104a, 104b. First vertical strut 110a, first outer upper strut 140a, first inner upper strut 142a, first central strut 130a and first outer lower strut 122a form a first generally kite-shaped cell 133a. Second vertical strut 110b, second outer upper strut 140b, second inner upper strut 142b, second central strut 130b and second outer lower strut 122b form a second generally kite-shaped cell 133b. First and second kite-shaped cells 133a, 133b are symmetric and opposite each other on stent section 107. Although the term "kite-shaped," is used above, it should be understood that such a shape is not limited to the exact geometric definition of kite-shaped. Outflow inverted V 114, first inner upper strut 142a and second inner upper strut 142b form upper cell 134. Upper cell 134 is generally kite-shaped and axially aligned with diamond cell 128 on stent section 107. It should be understood that, although designated as separate struts, the various struts described herein may be part of a single unitary structure as noted above. However, in other embodiments, stent 100 need not be formed as an integral structure and thus the struts may be different structures (or parts of different structures) that are coupled together.

Fig. 1C illustrates a schematic view of a stent section 207 according to an alternate embodiment of the disclosure. Unless otherwise stated, like reference numerals refer to like elements of above-described stent 100 but within the 200-series of numbers. Stent section 207 is substantially similar to stent section 107, including inflow nodes 202a, 202b, vertical struts 210a, 210b, first and second inflow inverted V's 220a, 220b and outflow nodes 204a, 204b. The structure of stent section 207 departs from that of stent section 107 in that it does not include an outflow inverted V. The purpose of an embodiment having such structure of stent section 207 shown in Fig. 1C is to reduce the required force to expand the outflow end 203 of the stent 200, compared to stent 100, to promote uniform expansion relative to the inflow end 201. Outflow nodes 204a, 204b are connected by a properly oriented V formed by first inner upper strut 242a, upper node 245 and second inner upper strut 242b. In other words, struts 242a, 242b may form a half diamond cell 234, with the open end of the half-cell oriented toward the outflow end 203. Half diamond cell 234 is axially aligned with diamond cell 228. Adding an outflow inverted V coupled between outflow nodes 204a, 204b contributes additional material that increases resistance to modifying the stent shape and requires additional force to expand the stent. The exclusion of material from outflow end 203 decreases resistance to expansion on outflow end 203, which may promote uniform expansion of inflow end 201 and outflow end 203. In other words, the inflow end 201 of stent 200 does not include continuous circumferential structure, but rather has mostly or entirely open half-cells with the open portion of the half-cells oriented toward the inflow end 201, whereas most of the outflow end 203 includes substantially continuous circumferential structure, via struts that correspond with struts 140a, 140b. All else being equal, a substantially continuous circumferential structure may require more force to expand compared to a similar but open structure. Thus, the inflow end 101 of stent 100 may require more force to radially expand compared to the outflow end 103. By omitting inverted V 114, resulting in stent 200, the force required to expand the outflow end 203 of stent 200 may be reduced to an amount closer to the inflow end 201.

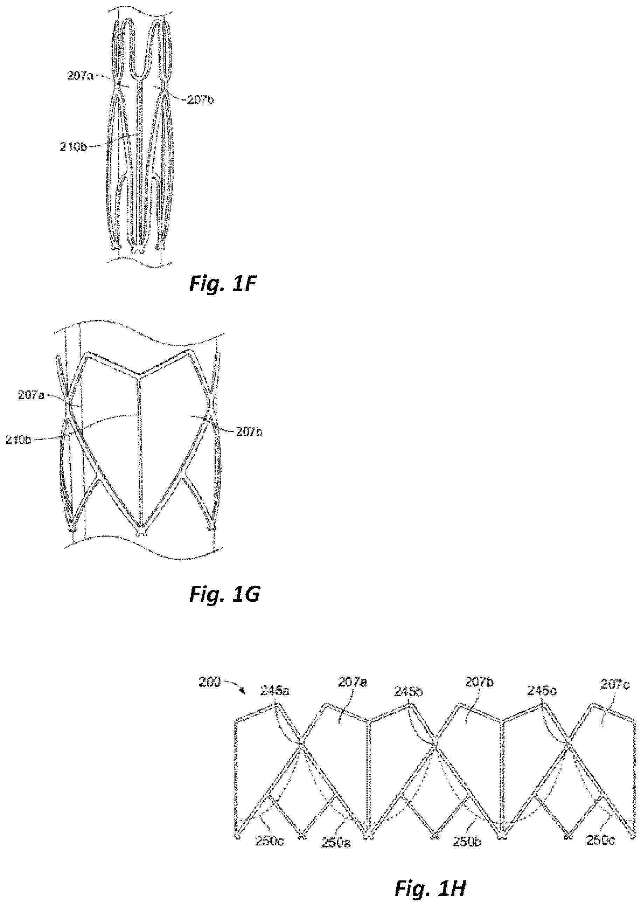

Fig. 1D shows a front view of stent section 207 in a collapsed state and Fig. 1E shows a front view of stent section 207 in an expanded state. It should be understood that stent 200 in Figs. 1D-E is illustrated with an opaque tube extending through the interior of the stent, purely for the purpose of helping illustrate the stent, and which may represent a balloon over which the stent section 207 is crimped. As described above, a stent comprises three symmetric sections, each section spanning about 120 degrees around the circumference of the stent. Stent section 207 illustrated in Figs. 1D-E is defined by the region between vertical struts 210a, 210b. Stent section 207 is representative of all three sections of the stent. Stent section 207 has an arcuate structure such that when three sections are connected, they form one complete cylindrical shape. Figs. 1F-G illustrate a portion of the stent from a side view. In other words, the view of stent 200 in Figs. 1F-G is rotated about 60 degrees compared to the view of Figs. 1D-E. The view of the stent depicted in FIGS. 1F-G is centered on vertical strut 210b showing approximately half of each of two adjacent stent sections 207a, 207b on each side of vertical strut 210b. Sections 207a, 207b surrounding vertical strut 210b are mirror images of each other. Fig. 1F shows stent sections 207a, 207b in a collapsed state whereas Fig. 1G shows stent sections 207a, 207b in an expanded state.

Fig. 1H illustrates a flattened view of stent 200 including three stent sections 207a, 207b, 207c, as if the stent has been cut longitudinally and laid flat on a table. As depicted, sections 207a, 207b, 207c are symmetric to each other and adjacent sections share a common vertical strut. As described above, stent 200 is shown in a flattened view, but each section 207a, 207b, 207c has an arcuate shape spanning 120 degrees to form a full cylinder. Further depicted in Fig. 1H are leaflets 250a, 250b, 250c coupled to stent 200. However, it should be understood that only the connection of leaflets 250a-c is illustrated in Fig. 1H. In other words, each leaflet 250a-c would typically include a free edge, with the free edges acting to coapt with one another to prevent retrograde flow of blood through the stent 200, and the free edges moving radially outward toward the interior surface of the stent to allow antegrade flow of blood through the stent. Those free edges are not illustrated in Fig. 1H. Rather, the attached edges of the leaflets 250a-c are illustrated in dashed lines in Fig. 1H. Although the attachment may be via any suitable modality, the attached edges may be preferably sutured to the stent 200 and/or to an intervening cuff or skirt between the stent and the leaflets 250a-c. Each of the three leaflets 250a, 250b, 250c, extends about 120 degrees around stent 200 from end to end and each leaflet includes a belly that may extend toward the radial center of stent 200 when the leaflets are coapted together. Each leaflet extends between the upper nodes of adjacent sections. First leaflet 250a extends from first upper node 245a of first stent section 207a to second upper node 245b of second stent section 207b. Second leaflet 250b extends from second upper node 245b to third upper node 245c of third stent section 207c. Third leaflet 250c extends from third upper node 245c to first upper node 245a. As such, each upper node includes a first end of a first leaflet and a second end of a second leaflet coupled thereto. In the illustrated embodiment, each end of each leaflet is coupled to its respective node by suture. However, any coupling means may be used to attach the leaflets to the stent. It is further contemplated that the stent may include any number of sections and/or leaflets. For example, the stent may include two sections, wherein each section extends 180 degrees around the circumference of the stent. Further, the stent may include two leaflets to mimic a bicuspid valve. Further, it should be noted that each leaflet may include tabs or other structures (not illustrated) at the junction between the free edges and attached edges of the leaflets, and each tab of each leaflet may be coupled to a tab of an adjacent leaflet to form commissures. In the illustrated embodiment, the leaflet commissures are illustrated attached to nodes where struts intersect. However, in other embodiments, the stent 200 may include commissure attachment features built into the stent to facilitate such attachment. For example, commissures attachment features may be formed into the stent 200 at nodes 245a-c, with the commissure attachment features including one or more apertures to facilitate suturing the leaflet commissures to the stent. Further, leaflets 250a-c may be formed of a biological material, such as animal pericardium, or may otherwise be formed of synthetic materials, such as ultra-high molecular weight polyethylene (UHMWPE).

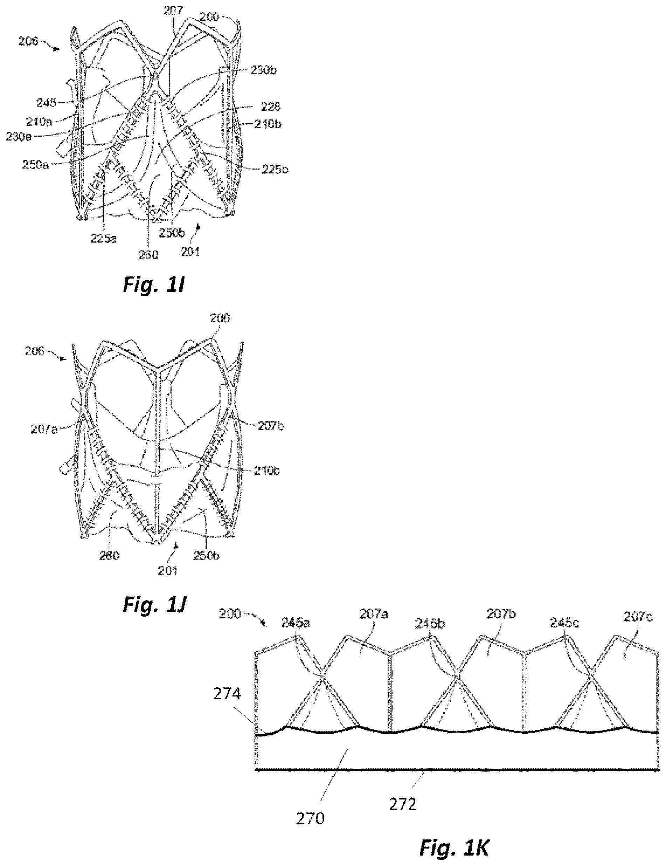

Figs. 1I-J illustrate prosthetic heart valve 206, which includes stent 200, a cuff 260 coupled to stent 200 (for example via sutures) and leaflets 250a, 250b, 250c attached to stent 200 and/or cuff 260 (for example via sutures). Prosthetic heart valve 206 is intended for use in replacing an aortic valve, although the same or similar structures may be used in a prosthetic valve for replacing other heart valves. Cuff 260 is disposed on a luminal or interior surface of stent 200, although the cuff could be disposed alternately or additionally on an abluminal or exterior surface of the stent. The cuff 260 may include an inflow end disposed substantially along inflow end 201 of stent 200. Fig. 1I shows a front view of valve 206 showing one stent portion 207 between vertical struts 210a, 210b including cuff 260 and an outline of two leaflets 250a, 250b sutured to cuff 260. Different methods of suturing leaflets to the cuff as well as the leaflets and/or cuff to the stent may be used, many of which are described in U.S. Pat. No. 9,326,856. In the illustrated embodiment, the upper (or outflow) edge of cuff 260 is sutured to first central node 225a, upper node 245 and second central node 225b, extending along first central strut 230a and second central strut 230b. The upper (or outflow) edge of cuff 260 continues extending approximately between the second central node of one section and the first central node of an adjacent section. Cuff 260 extends between upper node 245 and inflow end 201. Thus, cuff 260 covers the cells of stent portion 207 formed by the struts between upper node 245 and inflow end 201, including diamond cell 228. Fig. 1J illustrates a side view of stent 200 including cuff 260 and an outline of leaflet 250b. In other words, the view of valve 206 in Fig. 1J is rotated about 60 degrees compared to the view of Fig.1I. The view depicted in Fig. 1J is centered on vertical strut 210b showing approximately half of each of two adjacent stent sections 207a, 207b on each side of vertical strut 210b. Sections 207a, 207b surrounding vertical strut 210b are mirror images of each other. As described above, the cuff may be disposed on the stent's interior or luminal surface, its exterior or abluminal surface, and/or on both surfaces. A cuff ensures that blood does not just flow around the valve leaflets if the valve or valve assembly are not optimally seated in a valve annulus. A cuff, or a portion of a cuff disposed on the exterior of the stent, can help retard leakage around the outside of the valve (the latter known as paravalvular leakage or "PV" leakage). In the embodiment illustrated in Figs. 1I-J, the cuff 260 only covers about half of the stent 200, leaving about half of the stent uncovered by the cuff. With this configuration, less cuff material is required compared to a cuff that covers more or all of the stent 200. Less cuff material may allow for the prosthetic heart valve 206 to crimp down to a smaller profile when collapsed. It is contemplated that the cuff may cover any amount of surface area of the cylinder formed by the stent. For example, the upper edge of the cuff may extend straight around the circumference of any cross section of the cylinder formed by the stent. Cuff 260 may be formed of any suitable material, including a biological material such as animal pericardium, or a synthetic material such as UHMWPE.

As noted above, Figs. 1I-J illustrate a cuff 260 positioned on an interior of the stent 200. An example of an additional outer cuff 270 is illustrated in Fig. 1K. It should be understood that outer cuff 270 may take other shapes than that shown in Fig. 1K. The outer cuff 270 shown in Fig. 1K may be included without an inner cuff 260, but preferably is provided in addition to an inner cuff 260. The outer cuff 270 may be formed integrally with the inner cuff 260 and folded over (e.g., wrapped around) the inflow edge of the stent, or may be provided as a member that is separate from inner cuff 260. Outer cuff 270 may be formed of any of the materials described herein in connection with inner cuff 260. In the illustrated embodiment, outer cuff 270 includes an inflow edge 272 and an outflow edge 274. If the inner cuff 260 and outer cuff 270 are formed separately, the inflow edge 272 may be coupled to an inflow end of the stent 200 and/or an inflow edge of the inner cuff 260, for example via suturing, ultrasonic welding, or any other suitable attachment modality. The coupling between the inflow edge 272 of the outer cuff 270 and the stent 200 and/or inner cuff 260 preferably results in a seal between the inner cuff 260 and outer cuff 270 at the inflow end of the prosthetic heart valve so that any retrograde blood that flows into the space between the inner cuff 260 and outer cuff 270 is unable to pass beyond the inflow edges of the inner cuff 260 and outer cuff 270. The outflow edge 274 may be coupled at selected locations around the circumference of the stent 200 to struts of the stent 200 and/or to the inner cuff 260, for example via sutures. With this configuration, an opening may be formed between the inner cuff 260 and outer cuff 270 circumferentially between adjacent connection points, so that retrograde blood flow will tend to flow into the space between the inner cuff 260 and outer cuff 270 via the openings, without being able to continue passing beyond the inflow edges of the cuffs. As blood flows into the space between the inner cuff 260 and outer cuff 270, the outer cuff 270 may billow outwardly, creating even better sealing between the outer cuff 270 and the native valve annulus against which the outer cuff 270 presses. The outer cuff 270 may be provided as a continuous cylindrical member, or a strip that is wrapped around the outer circumference of the stent 200, with side edges, which may be parallel or non-parallel to a center longitudinal axis of the prosthetic heart valve, attached to each other so that the outer cuff 270 wraps around the entire circumference of the stent 200.

The stent may be formed from biocompatible materials, including metals and metal alloys such as cobalt chrome (or cobalt chromium) or stainless steel, although in some embodiments the stent may be formed of a shape memory material such as nitinol or the like. The stent is thus configured to collapse upon being crimped to a smaller diameter and/or expand upon being forced open, for example via a balloon within the stent expanding, and the stent will substantially maintain the shape to which it is modified when at rest. The stent may be crimped to collapse in a radial direction and lengthen (to some degree) in the axial direction, reducing its profile at any given cross-section. The stent may also be expanded in the radial direction and foreshortened (to some degree) in the axial direction.

The prosthetic heart valve may be delivered via any suitable transvascular route, for example including transapically or transfemorally. Generally, transapical delivery utilizes a relatively stiff catheter that pierces the apex of the left ventricle through the chest of the patient, inflicting a relatively higher degree of trauma compared to transfemoral delivery. In a transfemoral delivery, a delivery device housing the valve is inserted through the femoral artery and threaded against the flow of blood to the left ventricle. In either method of delivery, the valve may first be collapsed over an expandable balloon while the expandable balloon is deflated. The balloon may be coupled to or disposed within a delivery system, which may transport the valve through the body and heart to reach the aortic valve, with the valve being disposed over the balloon (and, in some circumstance, under an overlying sheath). Upon arrival at or adjacent the aortic valve, a surgeon or operator of the delivery system may align the prosthetic valve as desired within the native valve annulus while the prosthetic valve is collapsed over the balloon. When the desired alignment is achieved, the overlying sheath, if included, may be withdrawn (or advanced) to uncover the prosthetic valve, and the balloon may then be expanded causing the prosthetic valve to expand in the radial direction, with at least a portion of the prosthetic valve foreshortening in the axial direction.

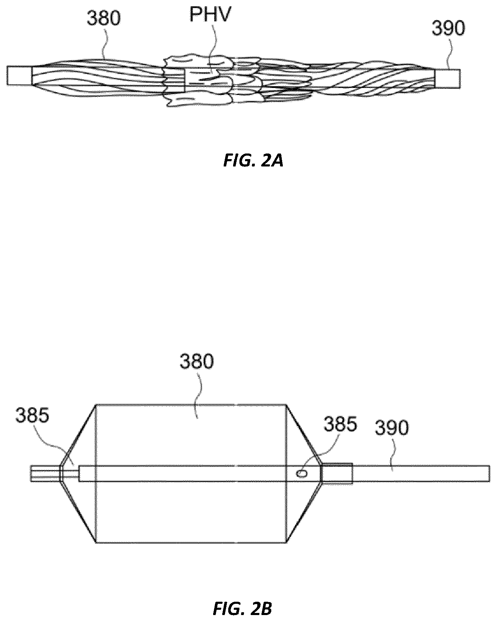

Referring to Fig. 2A, an example of a prosthetic heart valve PHV, which may include a stent similar to stents 100 or 200, is shown crimped over a balloon 380 of a balloon catheter 390 while the balloon 380 is in a deflated condition. It should be understood that other components of the delivery device, such as a handle used for steering and/or deployment, as well as a syringe for inflating the balloon 380, are omitted from Figs. 2A-B. The prosthetic heart valve PHV may be delivered intravascularly, for example through the femoral artery, around the aortic arch, and into the native aortic valve annulus, while in the crimped condition shown in Fig. 2A. Once the desired position is obtained, fluid may be pushed through the balloon catheter 390 to inflate the balloon 380, as shown in Fig. 2B. Fig. 2B omits the prosthetic heart valve PHV, but it should be understood that, as the balloon 380 inflates, it forces the prosthetic heart valve PHV to expand into the native aortic valve annulus (although it should be understood that other heart valves may be replaced using the concepts described herein). In the illustrated example, fluid flows from a syringe (not shown) into the balloon 380 through a lumen within balloon catheter 390 and into one or more ports 385 located internal to the balloon 380. In the particular illustrated example of Fig. 2B, a first port 385 may be one or more apertures in a side wall of the balloon catheter 390, and a second port 385 may be the distal open end of the balloon catheter 390, which may terminate within the interior space of the balloon 380.

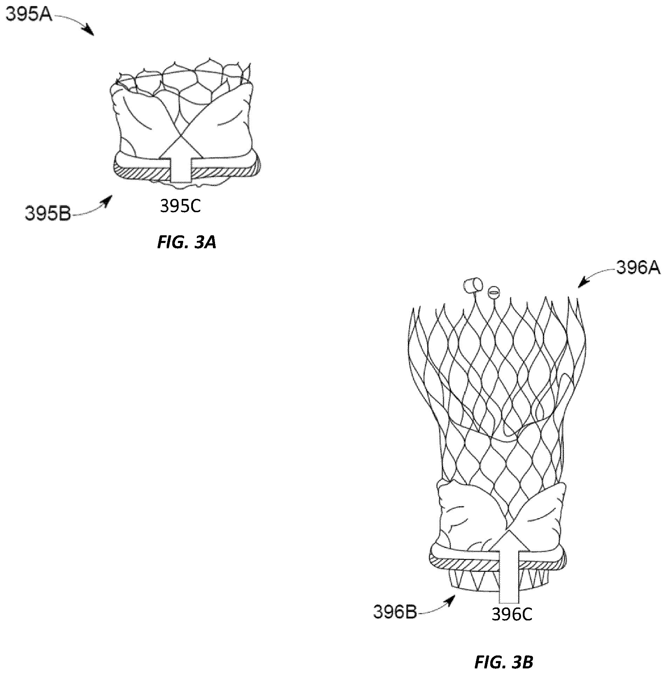

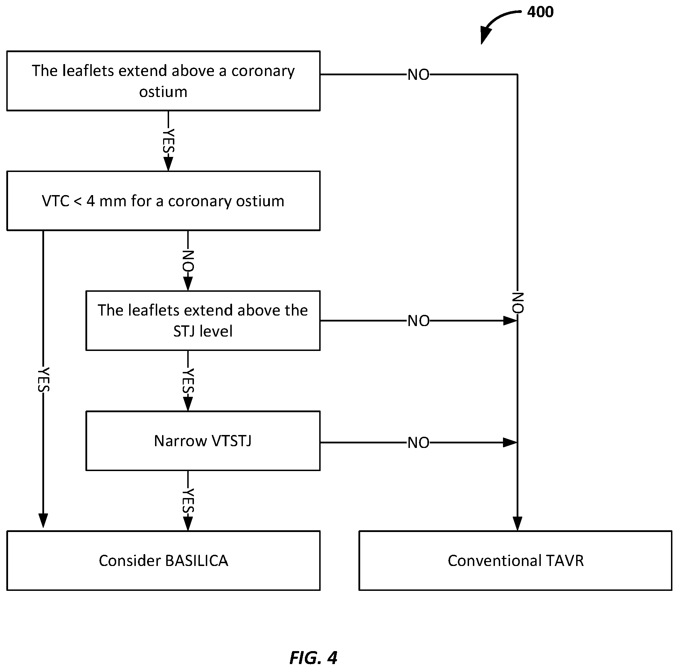

The present disclosure provides several systems, devices and methods to cut, lacerate, separate or notch a prosthetic or native valve leaflet. In Fig. 3A, a valve-in-valve procedure results in a first prosthetic heart valve 395A disposed inside a second prosthetic heart valve 395B. Optionally, a gap 395C is formed within the lacerated leaflet in a BASILICA procedure. Likewise, in Fig. 3B, a valve-in-valve procedure results in a prosthetic heart valve 396A disposed inside valve 396B, and a gap 396C may be formed. Through the leaflet gap, flow to the coronary sinus and arteries may be maintained as well as access to the coronary arteries for future interventions (e.g., if a patient has a heart attack and needs a coronary stent placed). While the remainder of the instant disclosure references a BASILICA procedure, the instruments, tools, system and methods described may also be useful for a LAMPOON procedure, which is similar to BASILICA but performed on the mitral leaflets. Not every valve-in-valve or implantation will require a BASILICA procedure. Instead, Figs. 4-5 illustrate a flow chart for assuming the risk for coronary obstruction and illustrations of several types of anatomies that may require a BASILICA procedure. In Fig. 4, assessment of potential coronary obstruction begins with observing the relationship between the coronary ostia and leaflet commissures. If the coronary ostia originate above the commissures, the leaflet may not reach and cover the coronary artery. Alternatively, if the leaflet extends above a coronary ostium, then the virtual transcatheter heart valve to coronary ostium distance (VTC) may be measured. If the VTC is less than 4 mm, then the BASILICA procedure should be considered. However, if the VTC is >4 mm, then the operator should check the risk for sinotubular junction (STJ)-inflow obstruction by evaluating the relationship between the sinotubular junction and the leaflet commissures. If the virtual transcatheter heart valve to sinotubular junction distance (VTSTJ) is relatively small, then a BASILICA procedure should be considered. However, there are insufficient data to suggest a high risk for obstruction in cases with a narrow VTSTJ. The operator also needs to assess leaflet bulkiness, and the position of stent posts in case they are in front of a coronary ostium. Thus, the relative position of the leaflet to the coronary ostium and its relationship to the sinotubular junction may affect whether a BASILICA procedure is desirable.

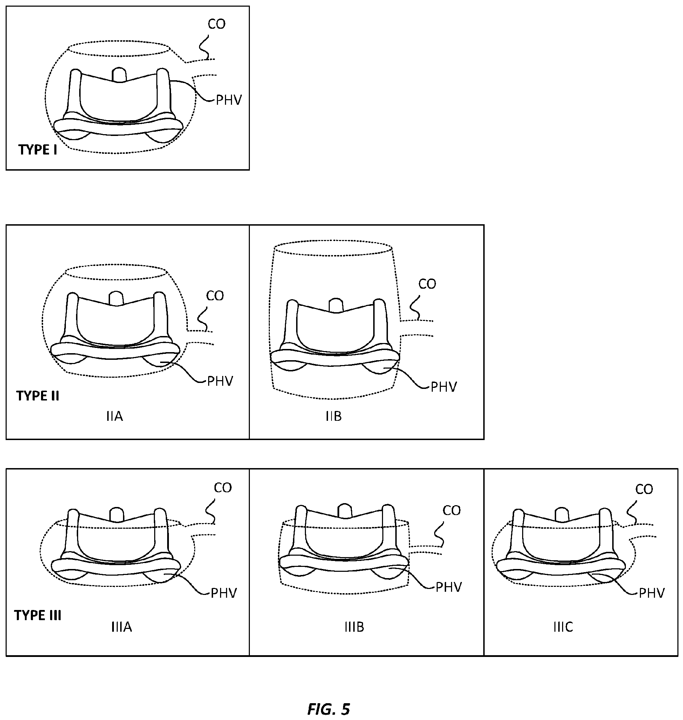

As shown in Fig. 5, the patient anatomy will be relevant and several examples are shown where a BASILICA procedure would be useful. Specifically, there are three main types of types of anatomical relations between the aortic root and the coronary ostium, and these may be relevant to defining the risks of coronary obstruction.

One example of Type I includes anatomies in which the coronary ostium "CO" lies above the top of the native or prosthetic leaflet of a prosthetic heart valve PHV. In a second example, labeled Type II, the coronary ostium "CO" may lie below the top of the leaflet. If the sinus is wide as shown on the left in quadrant IIA, then coronary obstruction has a lower chance of occurring. However, if the sinus is effaced as shown in the figure on the right in quadrant IIB, then coronary obstruction may occur. Type III includes situations where the implanted leaflets extend above the sinotubular junction (STJ) when deflected, for example, in supra-annular transcatheter valves. Three examples are provided for Type III and these includes conditions of a wide sinus and STJ (shown on the left in quadrant IIIA) with a lower chance of obstruction, and effaced sinuses (middle figure in quadrant IIIB) and narrow sinotubular junction (right figure in quadrant IIIC), which have a greater risk for coronary obstruction.

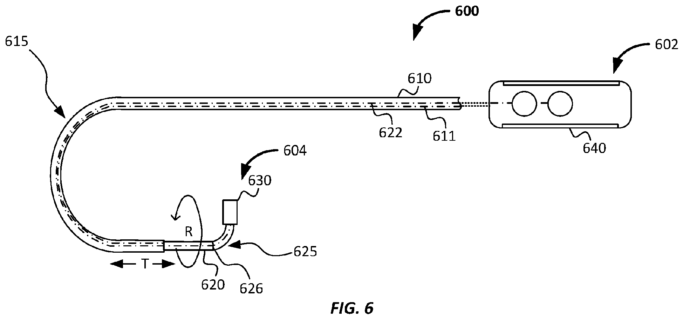

Fig. 6 illustrates a general overview of a medical instrument 600 for performing a BASILICA procedure. Generally, instrument 600 may extend between a proximal end 602 and a distal end 604. Instrument 600 may include a flexible outer sheath 610, a steerable inner catheter 620 at least partially disposed within the sheath, and a jaw assembly 630 at the distal tip of the inner catheter for manipulating and/or lacerating a leaflet. As a general overview, jaw assembly 630 may be used to grasp the leaflet during the procedure. The inner catheter and the jaw assembly may be steered and actuated via a handle 640 by the operator. In some examples, within the jaw assembly 630 is a cutting tool that facilitates laceration of the leaflet once positioning has been confirmed. This cutting tool may be mechanical (e.g., one or more sharp blades) or electrical (e.g., wire with electrosurgical generator) in nature and each of these variations will be described in greater detail below. The physician may perform BASILICA with instrument 600 by navigating the sheath toward the diseased aortic valve, grasping a leaflet under the left coronary, right coronary, or both, using x-ray and/or echo imaging, activating the cutting mechanism, and pulling back on the catheter until the leaflet has been cut through. In at least some examples, the sheath 610 and/or the inner catheter 620 may be flexible or deflectable to bend and pass through the aortic arch as shown with arrow 615, and the inner catheter 620 may also be deflectable in the annular plane as shown with arrow 625. To aid in the deflection in the annular plane, inner catheter 620 may include a deflecting member or hinge point 626 proximal to jaw assembly 630 to achieve a bend of approximately 90 degrees as shown. Inner catheter 620 may also be translateable as shown with arrow "T" and/or rotatable as shown with arrow "R" with respect to sheath 610.

In some examples, the outer sheath 610 is long enough to cross the aortic arch via transfemoral access. This may allow the physician to easily track the device to the annulus and allow navigation anteriorly and/or posteriorly within the annulus. One or more pull wires 611 may couple to the distal end of the sheath 610 and extend toward the handle 640 to allow the physician to control the sheath (e.g., to actively flex or bend it through the aortic arch). Within outer sheath 610, the inner catheter 620 may also easily track the anatomy and translate and/or rotate independently within the sheath 610. The deflectable section or hinge point 626 at arrow 625 may allow the jaw assembly to deflect parallel to the annular plane for leaflet grasping. In some examples, inner catheter 620 has one or more pull wires 622 running through it from its distal end to handle 640 that allow the physician to actuate the jaws from the handle 640.

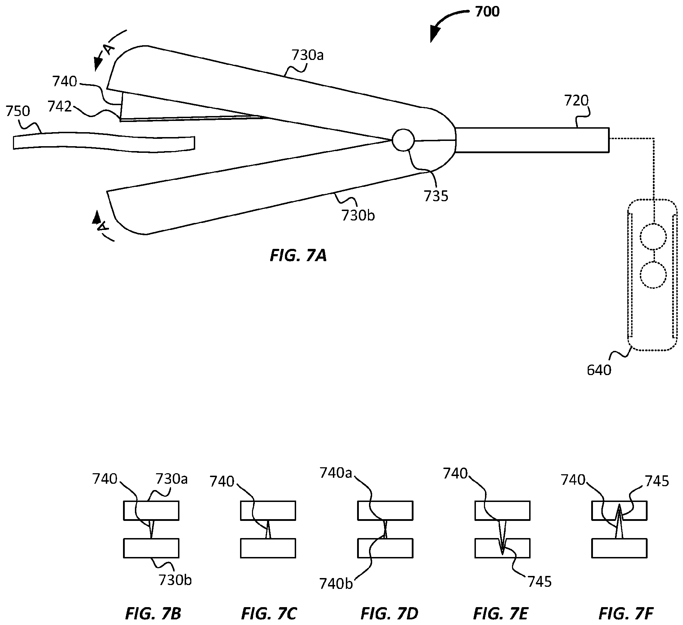

Turning to Fig. 7A, one example of a jaw assembly 700 will be described, the jaw assembly being configured for mechanical laceration. In this example, jaw assembly 700 generally includes a pair of opposing jaws 730a,730b that can articulate and/or pivot about a pin 735 to achieve open and closed conditions to grasp a leaflet or tissue by closing in the direction of arrows "A". Jaws 730a,730b may be directly attached to inner catheter 720 as shown. In some examples, jaws 730a,730b are made from metal or a polymer, and can be smooth or textured to engage with the tissue (e.g., diseased tissue). The jaws may engage tightly with tissue, or have a gap in the closed position to allow the jaws to slide over the tissue when cutting. In the example shown, a blade 740 is affixed to the upper jaw 730a, the blade 740 having a sharp cutting edge 742. Some alternatives are possible as shown in Figs. 7B-F. For example, a blade 740 may be affixed only to the upper jaw (Fig. 7B) or only the lower jaw (Fig. 7C). Two opposing blades 740 may be affixed to both jaws and oppose one another (Fig. 7D). The blade(s) may oppose a flat inner surface of the jaw, or a receiving slot 745 cut into the opposing lower jaw (Fig. 7E) or upper jaw (Fig. 7F). The blade 740 may also be recessed into the jaw and actuated using an independent control wire to allow leaflet grasping and laceration to be performed independently. The jaw assembly 730 and/or blade 740 actuation may be controlled at the handle, together or independently, and may be performed with or without proximal tension on the device to cut a tissue 750.

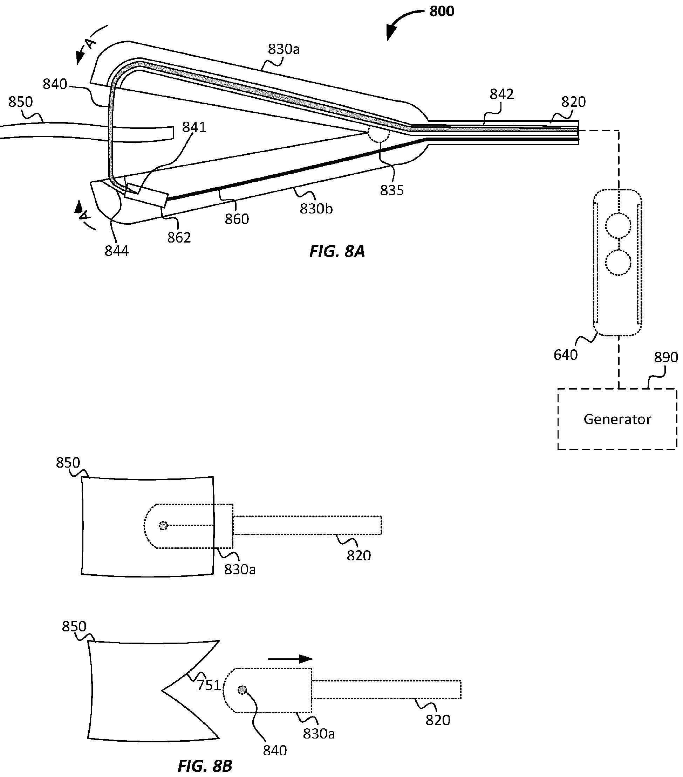

In an alternative embodiment, in accordance with the present claimed invention, a second example of a jaw assembly 800 will be described, the jaw assembly being configured for electrosurgical laceration. In this example, jaw assembly 800 generally includes a pair of opposing jaws 830a,830b that are pivotable about a pin 835 to achieve open and closed conditions to grasp a leaflet or tissue by closing in the direction of arrows "A". Jaws 830a,830b may be directly attached to inner catheter 820 as shown, and formed of the same material previously described. In the example shown, a channel 842 may be defined through inner catheter 820, and through the jaw assembly from a proximal end toward a distal end of first jaw 830a. A sharp, thin and flexible electrosurgical wire 840 may extend through the channel 842 and be translatable thereto, the wire 840 being coupled at a proximal end to electrical generator 890, and extend through the distal end of the jaw 830a where it exits toward the opposing jaw 830b. In at least some examples, electrosurgical wire 840 has a sharp piercing tip 841 that allows it to puncture through leaflet 850 before being passed toward the opposing jaw. The electrosurgical wire 840 may be oriented toward the second jaw 830b, and the second jaw 830b may include a straight or flared recess 844 for receiving the electrosurgical wire. A complementary conductive wire 860 may be electrically coupled to generator 890, extend through inner catheter 820 and/or jaw assembly 800. Specifically, conductive wire 860 may pass through second jaw 830b and terminate in a conductive receptacle 862 configured and arranged to receive, contact, and/or mate with electrosurgical wire 840 to form an electrical circuit as shown in Fig. 8A. Electrosurgical wire 840 and the proximal end of the conductive wire 860 may be connected, separately or together, to an electrosurgical generator 890 to energize the electrosurgical wire 840 when the circuit is completed, allowing it to cut through tissue and lacerate the leaflet 850 as the device is pulled proximally out of the patient, resulting in a straight laceration 751 that will form a V-shape as the leaflet stretches when a new valve frame is deployed inside of it as shown in the top view of leaflet 850 of Fig. 8B. The jaw assembly and/or electrosurgical wire actuation may be controlled at the handle, together or independently, and may be performed with or without proximal tension on the device to cut a tissue 850. It will be understood that generator 890 and/or handle may include one or more controls and/or buttons (e.g., an activation button for turning the device on/off) for selecting the appropriate voltage, frequency, power and temperature for safe delivery, cutting and removal of the medical instrument.

In use, the surgeon may perform a BASILICA procedure using either the mechanical or an electrosurgical variation. In either case, the medical instrument may be steered from the femoral artery up toward, and around, the aortic arch and down toward the aortic annulus via the handle. Beyond the aortic arch, the inner catheter may be extended further down toward the annulus and deflected or hinged toward the annular plane (See, Fig. 6). Once in position, the jaw assembly of the medical instrument may clamp down on the leaflet. In the mechanical variation, the jaws may bite onto the leaflet and one or more of the blades may lacerate the leaflet. In some examples, the biting of the jaws on the leaflet causes the sharp blade to form a slit, which is enough to form the needed laceration. In other examples, the operator may pull back on the medical instrument to extend the length of the laceration in the leaflet and the blades(s) may cut through the leaflet until it is released. As noted, an electrosurgical variation is possible. In this example, the procedure may be identical to that described, until the cutting step, where the electrosurgical wire would be advanced to lacerate the leaflet. Specifically, the electrosurgical wire may extend from the first jaw toward the second jaw, and may pierce the leaflet with its sharp tip and mate with the receptacle 862 to complete the electrical circuit. The first and second jaws may be brought together to gently secure the leaflet and the medical instrument may be carefully retracted. As the jaw assembly is retracted, the energized electrosurgical wire 840 may begin to burn or lacerate the leaflet to create a slit of a predetermined length. Once the wire 840 reaches the terminal end of the leaflet, the leaflet is released and the instrument may be removed from the wire.

It is to be understood that the embodiments described herein are merely illustrative of the principles and applications of the present disclosure. For example, a system may be battery-operated, or the handle and generator may be integrated. Additionally, a system may include both mechanical and electrical cutting elements. Moreover, certain components are optional, and the disclosure contemplates various configurations and combinations of the elements disclosed herein.

Although the invention herein has been described with reference to particular embodiments, it is to be understood that these embodiments are merely illustrative of the principles and applications of the present invention, as defined by the following claims.

## Claims

1. A medical instrument (600), comprising:
a flexible sheath (610) configured and sized to pass through a femoral artery;
an inner catheter (620; 820) at least partially disposed within the sheath (610) and rotatable and translatable relative thereto, the inner catheter (620; 820) having a deflectable section;
a pair of opposing jaws (830a; 830b) coupled to the inner catheter (620; 820), the pair of opposing jaws (830a; 830b) having an open condition and a closed condition;
a cutting mechanism disposed adjacent to at least one of the pair of opposing jaws (830a; 830b),
**characterized in that** the cutting mechanism comprises an electrosurgical wire (840) passing through the inner catheter (620; 820) to a generator (890), and extending through a first (830a) of the pair of opposing jaws (830a; 830b) and toward a second (830b) of the pair of opposing jaws (830a; 830b); and
wherein the medical instrument (600) further comprises a conductive wire (860) terminating in a conductive receptacle (862) disposed in the second of the pair of opposing jaws (830a; 830b), the conductive receptacle (862) being coupleable with the electrosurgical wire (840) to form an electrical circuit.

2. The medical instrument (600) of claim 1, wherein the deflectable section of the inner catheter (620; 820) is disposed proximal to the pair of opposing jaws (830a; 830b).

3. The medical instrument (600) of claim 1, wherein the deflectable section of the inner catheter (620; 820) includes a hinge point for annular plane deflection.

4. The medical instrument (600) of claim 1, further comprising a first channel defined within the first of the pair of opposing jaws (830a; 830b) for receiving the electrosurgical wire (840).

5. The medical instrument (600) of claim 4, wherein the first channel further extends through the inner catheter (620; 820).

6. The medical instrument (600) of claim 1, wherein transitioning the pair of opposing jaws (830a; 830b) to the closed condition serves to simultaneously couple the electrosurgical wire (840) to the conductive receptacle (826).

7. The medical instrument (600) of claim 1, wherein the electrosurgical wire (840) is coupleable to the conductive receptacle (826) independently from a condition of the pair of opposing jaws (830a; 830b).

8. The medical instrument (600) of claim 1, wherein the second (830b) of the pair of opposing jaws (830a; 830b) includes a recess (844) for receiving the electrosurgical wire (840).

9. The medical instrument (600) of claim 8, wherein the recess (844) is a flared recess.

10. The medical instrument (600) of claim 1, wherein the electrosurgical wire (840) is configured to lacerate tissue when energized.

11. The medical instrument (600) of claim 1, further comprising a handle (640), wherein the inner catheter (620; 820) and the pair of opposing jaws (830a; 830b) are actuated via the handle (640).

## Patentansprüche

1. Medizinisches Instrument (600), das Folgendes umfasst:
eine flexible Hülse (610), die zum Durchgang durch eine Oberschenkelarterie konfiguriert und dimensioniert ist;
einen inneren Katheter (620; 820), der zumindest teilweise innerhalb der Hülse (610) angeordnet ist und relativ dazu drehbar und verschiebbar ist, wobei der innere Katheter (620; 820) einen auslenkbaren Abschnitt aufweist;
ein Paar von gegenüberliegenden Backen (830a; 830b), die mit dem inneren Katheter (620; 820) verbunden sind, wobei das Paar von gegenüberliegenden Backen (830a; 830b) einen offenen Zustand und einen geschlossenen Zustand aufweist;
einen Schneidmechanismus, der benachbart zu mindestens einer des Paars von gegenüberliegenden Backen (830a; 830b) angeordnet ist,
**dadurch gekennzeichnet, dass** der Schneidmechanismus einen elektrochirurgischen Draht (840) umfasst, der durch den inneren Katheter (620; 820) zu einem Generator (890) durchgeht und sich durch eine erste (830a) des Paars von gegenüberliegenden Backen (830a; 830b) und in Richtung einer zweiten (830b) des Paars von gegenüberliegenden Backen (830a; 830b) erstreckt; und
wobei das medizinische Instrument (600) weiter einen leitenden Draht (860) umfasst, der in einer leitenden Aufnahmefassung (862) endet, die in der zweiten des Paars von gegenüberliegenden Backen (830a; 830b) angeordnet ist, wobei die leitende Aufnahmefassung (862) mit dem elektrochirurgischen Draht (840) verbindbar ist, um einen elektrischen Stromkreis zu bilden.

2. Medizinisches Instrument (600) nach Anspruch 1, wobei der auslenkbare Abschnitt des inneren Katheters (620; 820) proximal zu dem Paar von gegenüberliegenden Backen (830a; 830b) angeordnet ist.

3. Medizinisches Instrument (600) nach Anspruch 1, wobei der auslenkbare Abschnitt des inneren Katheters (620; 820) eine Gelenkstelle zur Auslenkung in einer ringförmigen Ebene einschließt.

4. Medizinisches Instrument (600) nach Anspruch 1, das weiter einen ersten Kanal umfasst, der innerhalb der ersten des Paars von gegenüberliegenden Backen (830a; 830b) zur Aufnahme des elektrochirurgischen Drahts (840) definiert ist.

5. Medizinisches Instrument (600) nach Anspruch 4, wobei sich der erste Kanal weiter durch den inneren Katheter (620; 820) erstreckt.

6. Medizinisches Instrument (600) nach Anspruch 1, wobei der Übergang des Paars von gegenüberliegenden Backen (830a; 830b) zu dem geschlossenen Zustand zur gleichzeitigen Verbindung des elektrochirurgischen Drahts (840) mit der leitenden Aufnahmefassung (826) dient.

7. Medizinisches Instrument (600) nach Anspruch 1, wobei der elektrochirurgische Draht (840), unabhängig von einem Zustand des Paars von gegenüberliegenden Backen (830a; 830b), mit der leitenden Aufnahmefassung (826) verbindbar ist.

8. Medizinisches Instrument (600) nach Anspruch 1, wobei die zweite (830b) des Paars von gegenüberliegenden Backen (830a; 830b) eine Aussparung (844) zur Aufnahme des elektrochirurgischen Drahts (840) einschließt.

9. Medizinisches Instrument (600) nach Anspruch 8, wobei die Aussparung (844) eine aufgeweitete Aussparung ist.

10. Medizinisches Instrument (600) nach Anspruch 1, wobei der elektrochirurgische Draht (840), bei Bestromung, zur Lazeration von Gewebe konfiguriert ist.

11. Medizinisches Instrument (600) nach Anspruch 1, das weiter einen Griff (640) umfasst, wobei der innere Katheter (620; 820) und das Paar von gegenüberliegenden Backen (830a; 830b) über den Griff (640) betätigt werden.

## Revendications

1. Instrument médical (600), comprenant :
une gaine flexible (610) configurée et dimensionnée pour passer à travers une artère fémorale ;
un cathéter interne (620 ; 820) au moins partiellement disposé dans la gaine (610) et rotatif et translatable par rapport à celle-ci, le cathéter interne (620, 820) ayant une section pouvant être défléchie ;
une paire de mâchoires opposées (830a ; 830b) couplées au cathéter interne (620 ; 820), la paire de mâchoires opposées (830a ; 830b) ayant une condition ouverte et une condition fermée ;
un mécanisme de coupe disposé adjacent à au moins l'une de la paire de mâchoires opposées (830a ; 830b),
**caractérisé en ce que** le mécanisme de coupe comprend un fil électrochirurgical (840) passant à travers le cathéter interne (602 ; 802) jusqu'à un générateur (890), et s'étendant à travers une première (830a) de la paire de mâchoires opposées (830a ; 830b) et vers une deuxième (830b) de la paire de mâchoires opposées (830a ; 830b) ; et
où l'instrument médical (600) comprend en outre un fil conducteur (860) se terminant dans un réceptacle conducteur (862) disposé dans la deuxième de la paire de mâchoires opposées (830a ; 830b), le réceptacle conducteur (862) pouvant être couplé au fil électrochirurgical (840) pour former un circuit électrique.

2. Instrument médical (600) selon la revendication 1, dans lequel la section pouvant être défléchie du cathéter interne (620 ; 820) est disposée proximale à la paire de mâchoires opposées (830a ; 830b).

3. Instrument médical (600) selon la revendication 1, dans lequel la section pouvant être défléchie du cathéter interne (620 ; 820) comprend un point charnière pour déflexion dans le plan annulaire.

4. Instrument chirurgical (600) selon la revendication 1, comprenant en outre un premier canal défini dans la première de la paire de mâchoires opposées (830a ; 830b) pour recevoir le fil électrochirurgical (840).

5. Instrument médical (600) selon la revendication 4, dans lequel le premier canal s'étend en outre à travers le cathéter interne (620 ; 820).

6. Instrument médical (600) selon la revendication 1, dans lequel faire passer la paire de mâchoires opposées (830a ; 830b) à la condition fermée sert aussi à coupler simultanément le fil électrochirurgical (840) au réceptacle conducteur (826).

7. Instrument médical (600) selon la revendication 1, dans lequel le fil électrochirurgical (840) peut être couplé au réceptacle conducteur (826) indépendamment d'une condition de la paire de mâchoires opposées (830a ; 830b).

8. Instrument médical (600) selon la revendication 1, dans lequel la deuxième (830b) de la paire de mâchoires opposées (830a ; 830b) comprend un évidement (844) pour recevoir le fil électrochirurgical (840).

9. Instrument médical (600) selon la revendication 8, dans lequel l'évidement (844) est un évidement évasé.

10. Instrument médical (600) selon la revendication 1, dans lequel le fil électrochirurgical (840) est configuré pour lacérer un tissu lorsque excité.

11. Instrument médical (600) selon la revendication 1, comprenant en outre une poignée (640), dans lequel le cathéter interne (620 ; 820) et la paire de mâchoires opposées (830a ; 830b) sont actionnés par la poignée (640).
